# EUROPEAN PATENT SPECIFICATION

(11) **EP 0 990 588 B1**
(45) Date of publication and mention of the grant of the patent: **07.05.2003**
(21) Application number: 98830575.1
(22) Date of filing: 30.09.1998
(51) Int. Cl.: B65C 9/18, B65H 35/08, B65H 20/12, A61F 13/15

(54) **Device for cutting from a web elements for laminating**
Vorrichtung zum Schneiden von Kaschierelementen aus Materialbahnen
Dispositif pour couper à partir d'une bande des éléments destinés à être laminés

(43) Date of publication of application: 05.04.2000
(73) Proprietor: Fameccanica. Data S.p.A., 66020 Sambuceto di S. Giovanni, Teatino (Chieti) (IT)
(72) Inventor: Pasqualoni, Paolo, 66020 Sambuceto di San Giovanni Teatino (IT)
(74) Representative: Bosotti, Luciano

(56) References cited:
- US-A- 3 903 768
- US-A- 5 486 253

## Description

The present invention concerns a device for cutting to length laminar elements. The invention has been developed with particular attention to its possible use in installations for the manufacture of hygienic and sanitary articles such as, for example, sanitary towels, diapers, etc.

One of the currently most widely used arrangements provides that these products are manufactured continuously or "in line", that is, from a plurality of strips or webs variously superimposed or coupled together to form a final strip or web from which the final individual articles are obtained (usually by means of cutting operations).

In order to achieve uninterrupted manufacturing processes, and therefore high velocity and efficiency, the individual elements constituting the product are coupled together while they travel at the same velocity. Since the elements to be coupled are characterised by different lengths and phases, specialised groups are present in the production machinery that have the function of first unwinding the material, then cutting elements of the desired length therefrom that are taken to the principal velocity of the line before coupling to the product being formed.

The accompanying drawings indicated 1A, 1B and 1C illustrate schematically the sequence of operation - in itself known - of a device for cutting laminar elements from a web.

The arrangement in question can be used, for example, to cut to length laminar elements (for example, labels etc.) from a web D of plastics material which unwinds from an associated supply reel, not explicitly illustrated in the drawings.

In order to be cut, the web material D is delivered at a given unwinding velocity, V1, to a cutting and take-off assembly constituted substantially by a rotating knife 1 and a so-called counter-knife 2.

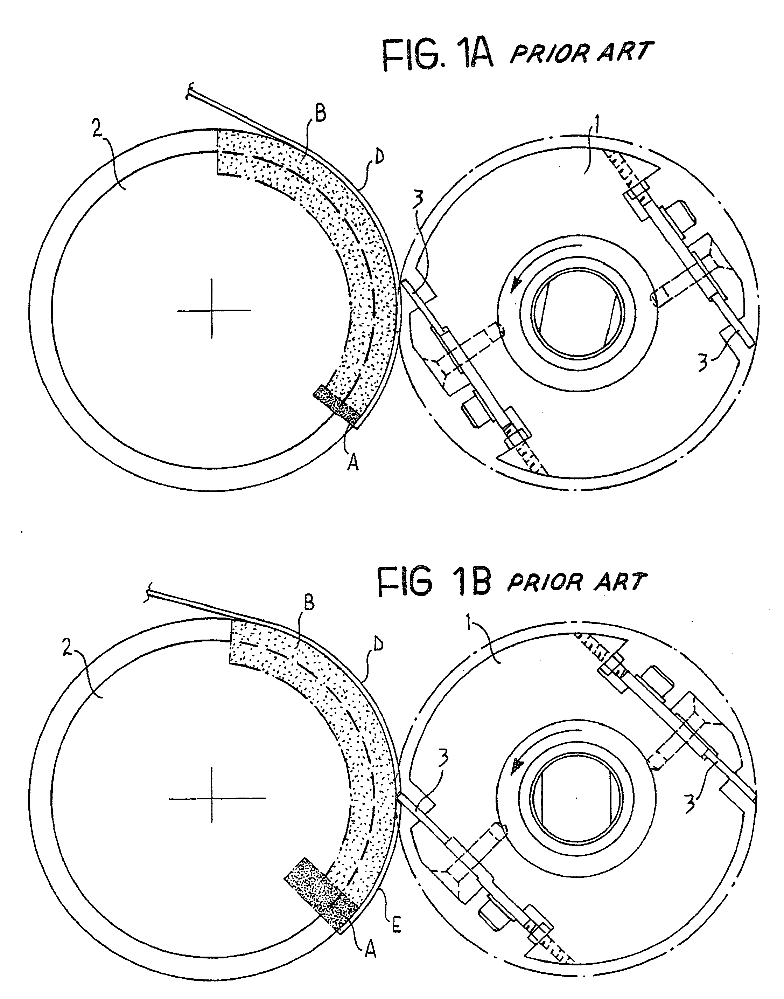

In the arrangement shown in Figures 1A to 1C, it is naturally assumed that the web D unwinds from the top downwards, and that the rotating knife 1, shown here with two blades 3, rotates in an anticlockwise direction while the counter-knife 2 rotates in a clockwise direction. It is also assumed that the angular velocity of the counter-knife 2 is such as to give rise to a tangential velocity of the surfacey (cladding) of the counter-knife 2 equal to V2, where V2 is generally greater than the unwinding velocity of the web D, V1.

In practice, the velocity V2 is chosen so that it is equal to the "line" velocity, that is, the velocity at which the labels E are successively applied to the product.

The unwinding velocity, indicated V1, is generally less - possibly significantly less - than V2 simply because the elements E cut from the web D are usually shorter, possibly significantly, than the products to which they are applied.

The counter-knife 2 has a pierced cladding surface in communication with its interior, where ducts leading to two vacuum lines (sources of sub-atmospheric pressure) are located. These latter establish a pressure gradient across the surface of the counter-knife 2 which holds the web D/the labels E in contact with the aforesaid cladding surface.

In particular, the two lines/sources of sub-atmospheric pressure are controlled so as to give rise to two levels of sub-atmospheric pressure known as "low vacuum" and "high vacuum", respectively. This denomination, which is currently used, is not at all correct from the physical point of view in that the "low" vacuum level corresponds in reality with an absolute pressure value greater than the absolute pressure value identified as the "high" level. Even as regards the actual values of these pressure levels, there are wide variations, even taking into account the other parameters in play (the open area/total area ratio of the cladding surface, etc.).

In practical terms, the distinction between the terms "low vacuum" and "high vacuum" comes simply from the effect on the web D.

When the web D comes into contact with regions of the cladding surface of the counter-knife 2 that are in the "low vacuum" state, the web D adheres to the cladding surface of the counter-knife 2, thus retaining the possibility of sliding or slipping with respect to this surface. On the other hand, when the web D is in contact with a region of the cladding surface of the counter-knife 2 in the "high vacuum" state, the counter-knife 2 exerts a decisive drive action on the web D, thus avoiding, at least substantially, the possibility of relative slipping.

The ducts within the counter-knife 2 that lead to the two vacuum lines are configured (according to various criteria) such that during the advancement through the tone in which the knife 1 acts, the web D itself interacts in succession with two regions of the cladding surface of the counter-knife 2. These two regions are, respectively, in a low vacuum condition (zone B) and a high vacuum condition (zone A). The spatial distribution of these regions varies as illustrated schematically in the sequence shown in Figures 1A to 1C.

Initially (Figure 1A), the free end of the web D - sometimes formed from the preceding cutting operation - advances into the space between the knife 1 and the counter-knife 2, extending so that practically its entire length is in contact with the low vacuum region B. The web D (delivered at the velocity V1) thus slides on the cladding surface of the counter-knife 2 which moves at a greater tangential velocity, V2.

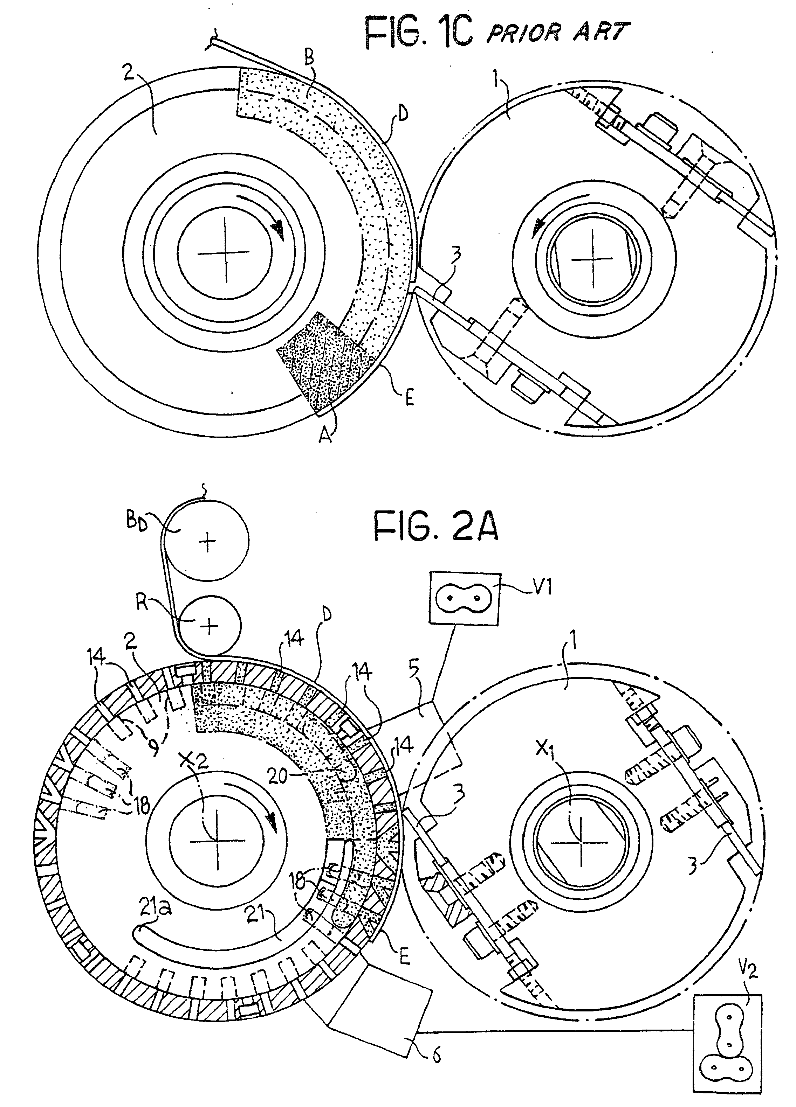

In order to avoid a situation in which the label E then formed (Figure 1B) by the blade 3 of the knife 1 that acts on the web D breaking up and - in particular - so that this label is, in fact, "captured" by the counter-knife 2 (making it advance at the line velocity V2, in exact synchronisation or "phasing" with respect to the flow of articles to which it is to be applied), the distal edge of the web D and then, gradually, the entire label E (see the sequence of Figures 1B and 1C) is gradually exposed to the region A which is in a high vacuum condition. Then, as can easily be seen in Figure 1A, exposure to the high vacuum zone A starts before the web D is cut by the blades 3 of the knife 1.

The known arrangement illustrated in Figures 1A-1C operates without difficulty even at high velocities, but is intrinsically limited in the case of elastic materials and/or materials that are particularly sensitive to stretching. In fact, as can be clearly seen from Figures 1A-1C, the material of the web D is, at least marginally, exposed contemporaneously both to the low vacuum region B and the high vacuum region A. Because of the difference between the velocity V2 (in practice, the velocity at which the high vacuum region A advances, which region captures the web D and pulls it therewith) and the processing velocity V1 of the web D, the web D itself is subjected to longitudinal traction and thus tends uncontrollably to develop an internal tension that is damaging to the process. This all carries the risk that the element E will be in some way damaged and/or transferred to the product while the element itself is subjected - in an undesirable manner - to stresses.

The above also applies substantially to the arrangement described in European Patent Application No. 98830154.5, in the name of the same Applicant. This describes a device for cutting to length laminar elements of a predetermined length, comprising web delivery means, means for receiving the web and the laminar elements formed therefrom, as well as cutting means interposed between the delivery means and the reception means, the delivery means being configured to enable the web to slip. Do-cuments such as US-A-3 903 768 or US-A-5 486 253 show that armehow similar arrangements used, e.g. for producing plastic-covered glass containers or labeling containers are intrinsically exposed to a speed difference and/or a tension being applied to the element being cut, which would not be sustainable in the case of elastic materials and/or materials which are particularly sensible to stretching. The requirement therefore exists to provide an arrangement which, inspired by the arrangement described above with reference to Figures 1A to 1C or by the substantially similar arrangement disclosed in US-A-3 903 768, after which the preamble of Claim 1 was patterned improves, the operation thereof by enabling its application to, for example, elastic materials.

According to the present invention, this object is achieved by virtue of a device having the further characteristics specifically referred to in the following claims.

Substantially, the principle on which the arrangement according to the invention is based is that of avoiding the web material being subjected before it is cut to the action of the high vacuum region which, by preventing the web from sliding, causes traction stress to develop. The high vacuum and its locking action are instead present only after the cutting action has been effected; the resulting element can be pulled by the counter-knife at the line velocity without this giving rise to stress on the web.

The invention will how be described, purely by way of non-limitative example, with reference to the accompanying drawings in which:
- Figures 1A to 1C, which represent the prior art, have already been described above;
- Figures 2A to 2C illustrate, in a way substantially analogous to that adopted for Figures 1A-1C, the characteristics of the arrangement according to the invention; and
- Figure 3 is an exploded perspective view of a possible embodiment of a counter-knife utilisable in a device according to the invention.

In Figures 2A to 2C, the same reference numbers are used that have already been used for the description of Figures 1A to 1C. Except for specific indications to the contrary, the elements referred to with the same reference numerals in Figures 1A to 1C and Figures 2A to 2C are to be considered as identical or equivalent to each other.

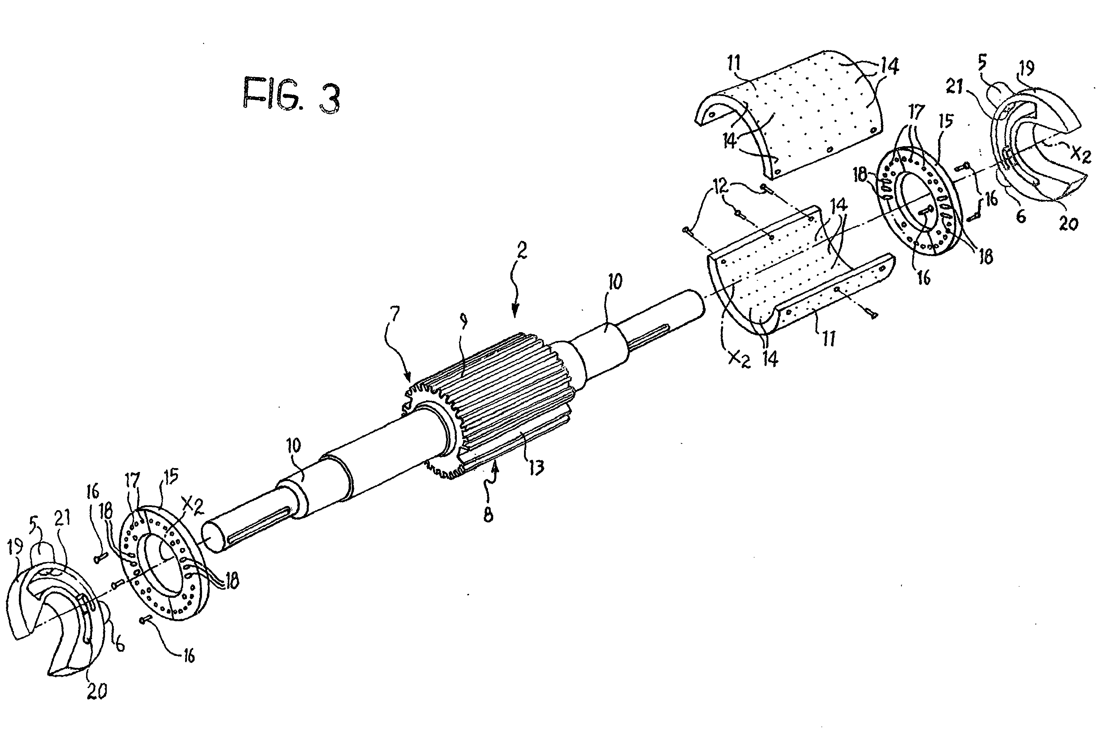

It will also be noted that the "low vacuum" and "high vacuum" sources have been identified in Figures 2A to 2C in the form of blocks V1 and V2, respectively. These sources lead via asspciated ducts or collectors 5, 6, into the counter-knife 2, as will be illustrated better below (with particular reference to Figure 3).

For the meaning of the terms "low vacuum" and "high vacuum", reference should be made to the introduction to the present description.

Also, as regards the general criteria of the operation for cutting the web D with the Knife 1 to form the labels E, the sequence of operations seen in Figures 2A to 2C does not differ substantially from that described above in relation to the prior art. It will also be noted that in Figures 2A to 2C show the source (reel) BD from which the web D unwinds (at the velocity V1, preferably with at least one interposed guide roller R).

The fundamental difference between the arrangement according to the invention and the arrangement according to the prior art lies substantially in the manner in which the "low vacuum" and "high vacuum" conditions alternate during the advancement of the web D and the cutting of the labels E.

In the arrangement illustrated in Figures 1A to 1C, this alternation is essentially in the form of a spatial succession, in the sense that during its advancement during the cutting operation, the web becomes involved with the high vacuum region A for a portion that increases in length from its distal end. This gives rise to longitudinal stress which is damaging, for example, to elastic materials.

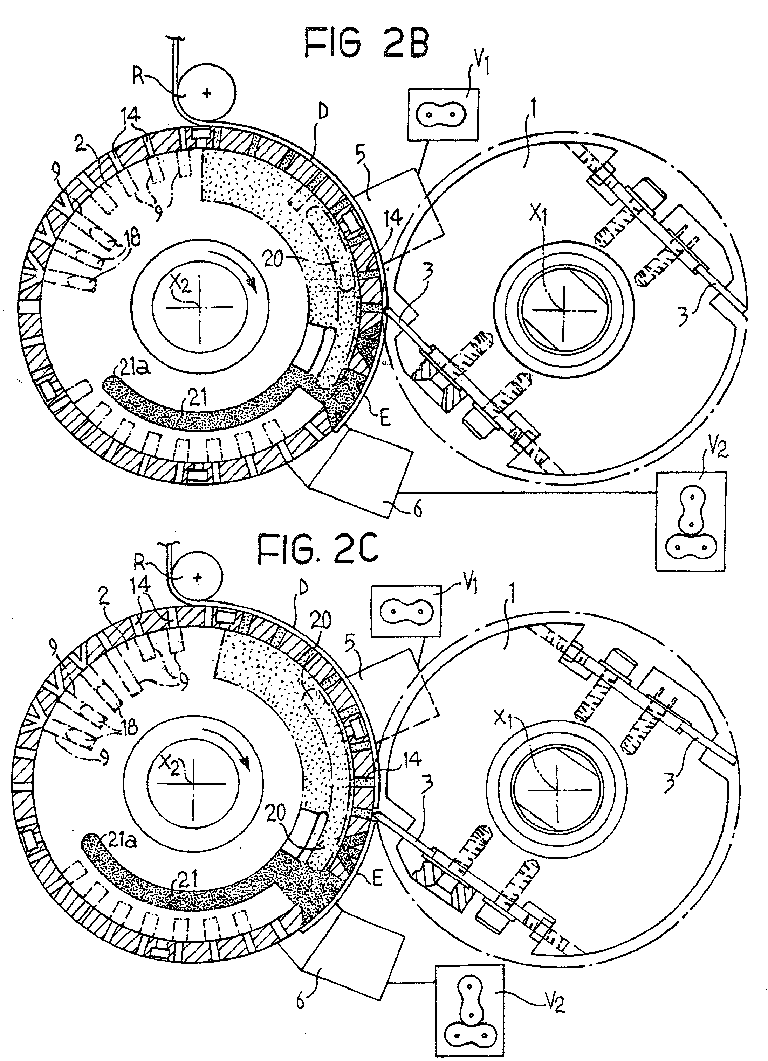

On the other hand, the arrangement according to the invention aims to alternate between the low and high vacuum conditions over time. In other words, the web D is subjected to the low vacuum conditions - and only to these - for the entire period preceding the cutting action by the knife 1 (Figure 2A). When the cutting action occurs (Figure 2B), the cut part of the web that forms the element or label E - and only this - is exposed to the action of the high vacuum in order to bring it to the line velocity (Figure 2C).

The arrangement according to the invention systematically avoids a situation in which a portion of web is at the same time still connected to the supply reel (at a velocity V1) and exposed to the high vacuum, and therefore dragged from the cladding surface of the counter-knife 2 at the velocity V2.

Figure 3 illustrates a possible embodiment of a counter-knife 2 capable of performing the function of alternating between the low and high vacuums as described above.

In the embodiment illustrated in Figure 3, the counter-knife 2 includes a rotor part and a stator part.

The rotor part includes a core 7 constituted by a cylindrical central body 8, the outer surface of which has a plurality of channels 9 orientated in the direction of the generatrix of the body 8 itself, and two axial extensions 10. The two extensions 10 are mounted in associated end bearings (not shown but of known type) and rotate the core 7 of the rotor about an associated principal axis X2 which, in fact, identifies the axis of rotation of the counter-knife 2.

Naturally, the assembly of the central body 8 and the extensions 10 can be formed as one piece or, according to an alternative that is preferable in many ways from the constructional point of view, as a central sleeve-like body having a channelled outer surface fitted and secured to a shaft the ends of which constitute the extensions 10. The adoption of a specific constructional arrangement is not, however, in itself a crucial element of the embodiment of the invention.

Two tile parts 11 are fitted about the central body 8, each having an angular extension of 180°. The tile parts 11 are held to the central, body 8 by known means (for example, by means of screws 12 which pass through the tile parts and engage corresponding radial holes in the body 8) and define the cylindrical cladding surface of the counter-knife 2. Apertures 14, usually in the form of circular holes, form intake openings in the cladding surface in question, through which the levels of sub-atmospheric pressure induced (in a manner illustrated below) in the channels 9 translate into corresponding pressure gradients intended to hold the web D in contact with the cladding surface of the counter-knife 2.

The rotor part of the counter-knife 2 further includes two generally annular end flanges 15 that can be formed in one or more pieces from a sealing material (for example, the material known under the commercial name "Ertalon").

The flanges in question are intended to be applied to the ends of the central body 8 by screws 16 that extend through corresponding holes in the flanges 15 to screw in the annular end surfaces of the body 8. All of this is in order to bring associated apertures 17 and 18 (usually in the form of holes) into exact alignment with the ends of the channels 9.

With the flanges 15 mounted on the body 8 to form, together with the tile parts 11, a rotatable drum, each of the chanziels 9 is aligned at one and/or the other of its ends with a first and/or a second associated aperture in one and/or the other of the flanges 15.

In the first instance, the aforesaid apertures are apertures 17 preferably realised in the form of a ring of holes arranged with respect to the central axis X2 of the counter-knife 2, at a radial distance practically corresponding to the radial distance that separates the channels 9 from the same axis X2.

Three generally slot-shaped apertures, indicated 18, are formed in each flange 15.

In practice, each aperture 18 can be seen as a kind of slot extending radially with respect to the flange 15. In particular, each aperture 18 has a radially external end practically aligned with the apertures 17 on the virtual circumference defined thereby, and a radially internal end that extends towards the central opening in the flange 15.

In Figure 3, two groups of three diametrically opposed apertures/slots 18 can be seen in each flange 15. This derives from the fact that the device illustrated in the accompanying drawings is configured such as to lead, on each rotation by 360° of the counter-knife 2, to the formation of two labels E in succession. This is consistent with the fact that the knife 1 illustrated has, in turn, two diametrically opposed blades 3 so that it is able to effect two successive cutting operations for each rotation by 360°.

Moreover, it is clear that the arrangement described here can be put into practice based on any so-called multiplicity value whatsoever comprising the unit value (one cutting intervention, and therefore one label E formed for each rotation by 360° of the knife 1 and the counter-knife 2): for clarity, the arrangement illustrated here can be seen as an arrangement with a multiplicity value equal to 2 (two labels formed for each rotation of the knife 1 and the counter-knife 2).

In any case, each of the two arcs of apertures 17 in the flanges 15, and the group of slots 18 preceding it can be seen essentially as an associated assembly of apertures of which a first group (those indicated 17) extends on a single radial level of the flange 15, while a second group (the slot-shape apertures indicated 18) extend on different radial levels of the flange 15.

The stator part of the counter-knife 2 is essentially constituted by two end manifolds 19 that are C-shape in the embodiment illustrated and have a radial notch that enables the mounting of the rotor part with the extensions 10 projecting from both sides with respect to the manifolds 19, with the consequent possibility of locating the associated support bearings outside the manifolds 19.

The vacuum lines, respectively, for the low vacuum (the source V1) and the high vacuum (the source V2), lead to these latter.

The manifolds 19 are essentially shaped bodies with internal through-holes that put the connectors 5 (low vacuum) and 6 (high vacuum) in communication with respective end apertures (distribution apertures) 20 and 21 which appear as associated arcuate paths on the faces of the manifolds 19 facing the flanges 15 and co-operating therewith in a relative sliding relationship with a substantial airtight seal, this being by virtue of the characteristics of the material forming the flanges 15.

In In particular (see Figures 2A to 2C in this regard), the distribution aperture 20 extends, with respect to the central axis X2 of the counter-knife 2, by a first radial distance corresponding substantially to the radial distance at which the apertures 17 are located, always with respect to this axis.

This means that all of the apertures 17, and the corresponding channels 9 of the body 8 that sometimes face the aperture 20 during the rotation of the counter-knife 2, are connected to the low vacuum source V1 via the connector 5.

On the other hand, the distribution aperture 21 extends at a second radial distance, always with respect to the axis X2 of the counter-knife 2, corresponding substantially to the distance at which the inner radial ends of the apertures/slots 18 are located.

Consequently, the channels 9 whose ends are located, together with the flanges 15 mounted against the central body 8, at the radially outer ends of the apertures/slots 18, can be connected (depending on the position reached at that time by the rotation of the counter-knife 2 about the axis X2) to the low vacuum source V1 as well as to the high vacuum source V2.

In particular, when the apertures 18 move along the arc of a circle corresponding to the angular extension of the aperture 20 (it is now supposed, for simplicity, that the apertures 20 and 21 extend along angularly separate arcs), the outer radial ends of the apertures 18 themselves put the connector 5, and therefore the low vacuum source V1, in communication with the corresponding channels 9 which are thus also located, like the other channels 9 aligned with the apertures 17, at the low vacuum level V1 of the source V1.

Conversely, when the apertures 18 correspond with the aperture 21, they (unlike the apertures 17 - which clearly do not have this possibility, given their angular arrangement) put the aperture 21, and therefore the connector 6 and the high vacuum source V2, in communication with the associated channels 9. Consequently, the channels 9 aligned with the apertures 18 - and only these channels - can be taken to the high vacuum condition.

Figures 2A to 2C show that, according to a currently preferred embodiment, the apertures 14 leading to the channels 9 communicating with the apertures 18 usually have a different shape from the other apertures 14 in the cladding surface of the counter-knife 2. For example, the cladding apertures leading to the apertures 18 can have a branched structure that enables a more regular and uniform distribution of the associated pressure gradient to be obtained.

Naturally, it is also possible (and this is exactly the arrangement preferably adopted in the invention) for the apertures 20 and 21 to extend along arcs of a circle that, although having different radii, superimpose on each other, at least marginally, from the angular point of view.

This fact is not influential as regards the apertures 17 and the channels 9 leading thereto: the apertures 17 always and therefore only connect with the aperture 20, and therefore only with the low vacuum line leading to the source V1.

Conversely, the apertures 18 - and therefore the channels 9 leading thereto - can, in the common parts of the apertures 20 and 21, be in contact both with the low vacuum line leading to the source V1 as well as the high vacuum line leading to the source V2. Under these conditions, the high vacuum line V2 becomes dominant so that, with an exception of a brief period deriving from the fact that the channels leading to the apertures 18 were previously connected to the low vacuum line, as soon as the connection (also) with the high vacuum line opens, the apertures 18 and the channels 9 leading thereto are brought to the high vacuum levels imposed by the source V2.

On the basis of the above, the angular extension of the apertures 20 and 21 is chosen, in a way co-ordinated with the position of the three slot-like apertures 18, so that while the knife 1 is not acting on the web D:
- the various apertures 14 leading to the channels 9 connected to the apertures 17 are connected by the aperture 20 and the connector 5 to the low vacuum line of the source V1, and
- the apertures 18 also, and thus the corresponding channels 9 and the apertures 14 leading thereto, are always and therefore only connected to the aperture 20 and the corresponding vacuum line.

Under these conditions (represented in Figure 2A), all of the apertures 14 against which the web D rests are connected to the low vacuum line of the source V1, and therefore to that referred to above as the "first region" in a low vacuum. Under these conditions, the web D advances at the delivery velocity V1, "skating", as it were, over the cladding surface of the counter-knife 2, which instead moves at the tangential velocity V2, which is greater than V1.

This operating condition lasts until the condition represented in Figure 2B is reached.

This is a condition in which, depending on its delivery velocity V1, the length of the terminal or free portion of the web D extending beyond the point of intervention of the knife 1 is equal to the length of the element or label E to be formed.

The positions of the apertures 18 and the aperture 21 (and also the number thereof: the presence of three apertures 18 as indicated in the accompanying drawings is purely by way of example and is certainly not a limitation of the invention) are chosen so that only from this moment the apertures 18, starting with that in the lowest position in Figure 2B, connect with the aperture 21 and, consequently, with the high vacuum line leading to the source V2, thus defining a corresponding "second region" at a high vacuum.

In particular, the location of the apertures 18 and the associated apertures 14 on the cladding surface of the counter-knife 2 is chosen so that the aperture or apertures 18 (and only these) that can connect with the high vacuum line (the source V2) lead to the apertures 14 on the cladding of the counter-knife 2, which are all downstream of the point at which the knife 1 has just cut the web D.

In this way, the element or label E just formed is firmly captured on the cladding surface of the counter-knife 2, and starts to move at the tangential velocity V2 for its application to the product.

Conversely, all of the apertures 14 co-operating with the portion of the web D upstream of the cutting point (which continued to advance at the unwinding velocity V1) are, and continue to be, all connected via the aperture 20 to the low vacuum line leading to the source V1.

In this way, while the label E that has just been cut is taken away from the counter-knife 2 at the velocity V2, held firmly on the cladding surface 14 leading to the apertures 18 (which are in the high vacuum state), the successive portion of web, intended to be cut again to form a new label, is held in contact in the counter-knife 2 by apertures 14 connected to the low vacuum line, which means that the web D can slide or slip freely with respect to the surface of the counter-knife.

Naturally, the terms "upstream" and "downstream", as utilised here, refer to the normal direction of advancement of the web D and the envisaged point of intervention of the knife 1. In the embodiment illustrated, this point is located on the line that, in Figures 2A to 2C, joins the path of the axis X2 of the counter-knife 2 with the path of the equivalent axis, indicated X1, of the knife 1.

From Figures 2A to 2C, in particular Figure 2C, it can be noted that the aperture 21 is interrupted at a certain point, indicated 21A, in such a way as to interrupt the connection between the apertures 18 that advance together with the said terminal ends of the aperture 21 due to the rotation of the counter-knife 2. The interruption of the connection to the high vacuum line thus obtained does not usually provide for the contemporaneous re-establishment of the connection with the low vacuum line (a condition that is re-established only in a successive moment on the path of rotation of the apertures 18, when these latter locate the aperture 20). Downstream of the point indicated 21A, the apertures 18 are practically separated from each other by the other vacuum line, that is, at approximately atmospheric pressure. On reaching this angular position, the element or label E is no longer held by the vacuum to the cladding surface of the counter-knife 2 and can therefore be transferred (directly or with the interposing of a further rotating body, for example, structurally similar to the counter-knife 2) to the final product.

Naturally, the principal of the invention remaining the same, the details of construction and the embodiments can be widely varied with respect to that described and illustrated without by this departing from the ambit of the present invention as defined in the following claims.

## Claims

1. A device for cutting laminar elements (E) of a given length from a web (D), comprising:
- a movable element (2) which moves at an associated velocity (V2) and co-operates in contact relationship with the said web (D) which advances at a delivery velocity (V1) less than the said velocity of the movable element (2);
- cutting means (1) that co-operate with the said movable element (2) to perform successive cutting interventions on the said web (D) to form the said laminar elements (E);
- first means (V1) for generating sub-atmospheric pressure to produce a first vacuum region in the said movable element (2), which holds the said web (D) in a sliding relationship on the said movable element (2); and
- second means (V2) for generating sub-atmospheric pressure to produce a second vacuum region in the said movable element (2), which holds the said web (D) on the said movable element in a drive relationship, wherein:
the said first (V1) and second (V2) means for generating sub-atmospheric pressure have associated means (20 and 21) for controlling the extension of the said first and second vacuum regions for the synchronised intervention with the said cutting means (1) so that, for each successive intervention of the cutting means (1):
- before the intervention of the cutting means (1), the portion of web (D) co-operating with the movable element (2) is exposed exclusively to the said first vacuum region; and
- after the intervention of the cutting means (1), the laminar element (E) formed by cutting is exposed to the second vacuum region, while the remaining part of the web (D) co-operating with the said movable element (2) remains exposed to the said first vacuum region,
- the said movable element (2) has a surface for co-operation with the said web (D) provided with holes (9) and brought to a level of sub-atmospheric pressure for co-operation with the said web; **characterised in that**:
- the said holes (9) have associated first (17) and second (18) groups of sub-atmospheric pressure distribution means, of which:
- the said first group (17) is exclusively connectable to the said first sub-atmospheric pressure generation means (V1) so that the respective holes (9) define exclusively the said first vacuum region, and
- the said second group (18) can be connected both to the said first (V1) as well as the second (V2) sub-atmospheric pressure generation means, so that the respective holes (9) can belong both to the said first vacuum region, as well as the second vacuum region, depending on the position reached by the said movable element.

2. A device according to Claim 1, **characterised in that** the said movable element (1) comprises a rotatable drum (7, 11, 15) having a pierced cladding surface (14).

3. A device according to Claim 2, **characterised in that** .the said control means include distribution apertures (20, 21) extending along angular paths that are at least partially separated along the path of rotation of the said drum (7, 11, 15).

4. A device according to Claim 3, **characterised in that** the said distribution apertures include at least one aperture (21) connected to the said second pressure generation means (V2) and which extend along an angular path having an end corresponding to the connection of the said second vacuum region with the said second sub-atmospheric pressure generation means (V2), the said end being located at the position reached by the said drum (7, 11, 15) at the moment in which the said cutting means (1) perform the said cutting intervention.

5. A device according to Claim 1 and Claim 2, **characterised in that**:
- the said holes (9) extend in a substantially axial direction with respect to the associated rotatable drum (2); and
- the said vacuum distribution means of the first and second groups are constituted by end apertures (17, 18) of the said drum (7, 11, 15), of which the apertures defining the said first group (17) of distribution means are located at a given radial distance from the main axis (X2) of the said drum (7, 11, 15), while the apertures defining the said second group (18) of distribution means extend radially for a certain distance with respect to the said main axis (X2) of the said drum (7, 11, 15).

6. A device according to Claim 3 and Claim 5, **characterised in that**:
- the said first distribution aperture (20) extends substantially to first radial distance from the main axis of the said drum (7, 11, 15), the said first distance being substantially equal to the said given radial distance at which the first apertures (17) are located;
- the said second distribution aperture (21) extends, with respect to the said main axis (X2) of the drum, to a second radial distance that is different from the said first radial distance; and
- the apertures (18) defining the said second group of distribution means extend radially with respect to the drum (7, 11, 15), at least for the portion between the said first and said second radial distances.

7. A device according to any of Claims 1, 6 or 7 **characterised in that** the said first (17) and second (18) groups of distribution means are provided in at least one terminal flange (15) associated with the said drum (7, 11, 15).

8. A device according to Claim 3 and Claim 7, **characterised in that** the said at least one terminal flange (15) is fixed in rotation with the said drum (7, 11, 15), while the said first (20) and second (21) distribution apertures are provided in at least one distribution element (19) located in a static position with respect to the said drum (7, 11, 15).

9. A device according to Claim 8, **characterised in that**:
- at least one axial support extension (10) which rotates about the said main axis (X2) is associated with the said rotating drum (7, 11, 15);
- the said at least one distribution element (19) has a hollow for the said at least one axial extension (10) to pass through.

10. A device according to Claim 8 or Claim 9, **characterised in that** the said at least one flange (12) is formed from a pneumatic sealing material able to form the airtight seal with the said at least one distribution element (19).

11. A device according to any preceding claim, **characterised in that** the said cutting means (1) include a rotary cutting element, the rotation movement of which is synchronised with the movement of the said movable element (2).

## Patentansprüche

1. Vorrichtung zum Schneiden blattförmiger Elemente (E) einer vorbestimmten Länge aus einer Materialbahn (D), die aufweist:
ein bewegbares Element (2), das sich mit einer zugeordneten Geschwindigkeit (V2) bewegt und berührend mit der Materialbahn (D) zusammenwirkt, die sich mit einer Zuführgeschwindigkeit (V1), die geringer als die Geschwindigkeit des bewegbaren Elementes (2) ist, vorwärts bewegt;
ein Schneidmittel (1), das mit dem bewegbaren Element (2) zusammenwirkt, um zum Erzeugen der blattförmigen Elemente (E) aufeinanderfolgende Schneideingriffe an der Materialbahn (D) durchzuführen;
eine erste Einrichtung (V1) zum Erzeugen eines Unterdruckes, um in dem bewegbaren Element (2) einen ersten Vakuumbereich zu erzeugen, der die Materialbahn (D) gleitend an dem bewegbaren Element (2) hält; und
eine zweite Einrichtung (V2) zum Erzeugen eines Unterdruckes, um in dem bewegbaren Element (2) einen zweiten Vakuumbereich zu erzeugen, der die Materialbahn (D) an dem bewegbaren Element hält, so daß diese angetrieben wird, wobei
die erste Einrichtung (V1) und die zweite Einrichtung (V2) zugeordnete Mittel (20 und 21) zum Erzeugen eines Unterdruckes zur Steuerung der räumlichen Ausdehnung des ersten und des zweiten Vakuumbereiches umfaßt, um einen synchronisierten Eingriff mit dem Schneidmittel (1) zu erzielen, so daß bei jedem der aufeinanderfolgenden Eingriffe des Schneidmittels (1)
vor dem Eingriff des Schneidmittels (1) nur derjenige Bereich der Materialbahn (D), der mit dem bewegbaren Element (2) zusammenwirkt, dem ersten Vakuumbereich ausgesetzt ist; und
nach dem Eingriff des Schneidmittels (1) das durch den Schneidvorgang erzeugte blattförmige Element (E) dem zweiten Vakuumbereich ausgesetzt ist, während der restliche Bereich der Materialbahn (D), der mit dem bewegbaren Element (2) zusammenwirkt, dem ersten Vakuumbereich ausgesetzt bleibt,
wobei das bewegbare Element (2) eine Oberfläche zum Zusammenwirken mit der Materialbahn (D) umfaßt, die Löcher (9) und ein Unterdruckniveau zum Zusammenwirken mit der Materialbahn aufweist; **dadurch gekennzeichnet, daß**
die Löcher (9) zugeordnete erste (17) und zweite (18) Gruppen von Unterdruckverteilungsmitteln aufweist, wobei
die erste Gruppe (17) ausschließlich mit der ersten Unterdruckerzeugungseinrichtung (V1) verbindbar ist, so daß die entsprechenden Löcher (9) ausschließlich den ersten Vakuumbereich definieren, und
die zweite Gruppe (18) sowohl mit der ersten (VI) als auch mit der zweiten (V2) Unterdruckerzeugungseinrichtung verbindbar ist, so daß die entsprechenden Löcher (9) abhängig von der Position des bewegbaren Elementes sowohl zu dem ersten Vakuumbereich als auch zu dem zweiten Vakuumbereich gehören können.

2. Vorrichtung nach Anspruch 1, **dadurch gekennzeichnet, daß** das bewegbare Element (2) eine drehbare Trommel (7, 11, 15) mit einer gelochten Hüllfläche (14) aufweist.

3. Vorrichtung nach Anspruch 2, **dadurch gekennzeichnet, daß** das Steuermittel Verteilungsöffnungen (20, 21) umfaßt, die sich entlang winkelförmig angeordneter Wege erstrecken und wenigstens teilweise entlang des Drehweges der Trommel (7, 11, 15) voneinander getrennt sind.

4. Vorrichtung nach Anspruch 3, **dadurch gekennzeichnet, daß** die Verteilungsöffnungen wenigstens eine Öffnung (21) umfassen, die mit der zweiten Druckerzeugungseinrichtung (V2) verbunden ist und sich entlang eines winkelförmig angeordneten Weges erstreckt, der ein Ende aufweist, das der Verbindung des zweiten Vakuumbereiches mit der zweiten Unterdruckerzeugungseinrichtung (V2) entspricht, wobei das Ende an derjenigen Position angeordnet ist, welche die Trommel (7, 11, 15) in dem Moment erreicht, in dem das Schneidmittel (1) den Schneideingriff durchführt.

5. Vorrichtung nach Anspruch 1 und 2, **dadurch gekennzeichnet, daß**
sich die Löcher (9) in einer im wesentlichen axialen Richtung hinsichtlich der zugehörigen drehbaren Trommel (2) erstrecken; und
die Vakuumverteilungsmittel der ersten Gruppe und der zweiten Gruppe durch Endöffnungen (17, 18) der Trommel (7, 11, 15) gebildet sind, wobei diejenigen Öffnungen, welche die erste Gruppe (17) von Verteilungsmitteln definieren, in einem vorbestimmten radialen Abstand von der Hauptachse (X2) der Trommel (7, 11, 15) angeordnet sind, während sich diejenigen Öffnungen, welche die zweite Gruppe (18) von Verteilungsmitteln definieren, über eine bestimmte Strecke radial hinsichtlich der Hauptachse (X2) der Trommel (7, 11, 15) erstrecken.

6. Vorrichtung nach Anspruch 3 und 5, **dadurch gekennzeichnet, daß**
sich die erste Verteilungsöffnung (20) im wesentlichen in einem ersten radialen Abstand von der Hauptachse der Trommel (7, 11, 15) erstreckt, wobei der erste Abstand im wesentlichen dem vorbestimmten radialen Abstand entspricht, in dem die ersten Öffnungen (17) angeordnet sind;
sich die zweite Verteilungsöffnung (21) hinsichtlich der Hauptachse (X2) der Trommel in eine zweiten radialen Abstand erstreckt, der von dem ersten radialen Abstand verschieden ist; und
sich die Öffnungen (18), welche die zweite Gruppe von Verteilungsmitteln definieren, radial hinsichtlich der Trommel (7, 11, 15) erstrecken, zumindest in dem Bereich zwischen dem ersten radialen Abstand und dem zweiten radialen Abstand.

7. Vorrichtung nach einem der Ansprüche 1, 6 oder 7, **dadurch gekennzeichnet, daß** die erste Gruppe (17) und die zweite Gruppe (18) von Verteilungsmitteln an wenigstens einem Endflansch (15) der Trommel (7, 11, 15) angeordnet sind.

8. Vorrichtung nach Anspruch 3 und 7, **dadurch gekennzeichnet, daß** der wenigstens eine Endflansch (15) drehfest an der Trommel (7, 11, 15) befestigt ist, während die ersten (20) und zweiten (21) Verteilungsöffnungen an wenigstens einem Verteilungselement (19) vorgesehen sind, das ortsfest hinsichtlich der Trommel (7, 11, 15) angeordnet ist.

9. Vorrichtung nach Anspruch 8, **dadurch gekennzeichnet, daß**
wenigstens ein axialer Halteansatz (10), der sich um die Hauptachse (X2) dreht, mit der Drehtrommel (7, 11, 15) verbunden ist; und
das wenigstens eine Verteilungselement (19) eine Öffnung aufweist, durch die sich der wenigstens eine axiale Halteansatz (10) erstreckt.

10. Vorrichtung nach Anspruch 8 oder 9, **dadurch gekennzeichnet, daß** der wenigstens eine Flansch (12) ein Druckluftdichtungsmaterial aufweist, mit dem eine luftdichte Dichtung zu dem wenigstens einen Verteilungselement (19) erzeugt werden kann.

11. Vorrichtung nach einem der vorhergehenden Ansprüche, **dadurch gekennzeichnet, daß** das Schneidmittel (1) ein drehbares Schneidelement aufweist, dessen Drehbewegung mit der Bewegung des bewegbaren Elementes (2) synchronisiert ist.

## Revendications

1. Dispositif de coupe d'éléments laminaires (E) de longueur déterminée dans une feuille continue (D), comprenant :
un élément mobile (2) qui se déplace à une vitesse associée (V2) et coopère en relation de contact avec la feuille continue (D) qui avance à une vitesse de distribution (V1) inférieure à la vitesse de l'élément mobile (2),
un dispositif de coupe (1) qui coopère avec l'élément mobile (2) pour exécuter des interventions de coupe successive sur la feuille continue (D) pour la formation des éléments laminaires (E),
un premier dispositif (V1) de création d'une dépression destinée à produire une première région de vide dans l'élément mobile (2), maintenant la feuille continue (D) en relation de glissement sur l'élément mobile (2), et
un second dispositif (V2) destiné à créer une dépression destinée à produire une seconde région de vide dans l'élément mobile (2) pour le maintien de la feuille continue (D) sur l'élément mobile en relation d'entraînement, dans lequel
le premier (V1) et le second (V2) dispositif de création d'une dépression ont un dispositif associé (20 et 21) destiné à régler l'étendue des première et seconde régions de vide pour l'intervention synchronisée avec le dispositif de coupe (1) si bien que, pour chaque intervention successive du dispositif de coupe (1) :
avant l'intervention du dispositif de coupe (1), la partie de feuille continue (D) qui coopère avec l'élément mobile (2) est exposée exclusivement à la première région de vide, et
après l'intervention du dispositif de coupe (1), l'élément laminaire (E) formé par coupe est exposé à la seconde région de vide alors que la partie restante de la feuille continue (D) coopérant avec l'élément mobile (2) reste exposée à la première région de vide,
l'élément mobile (2) a une surface destinée à coopérer avec la feuille continue (D) ayant des trous (9) et mise à un niveau de dépression permettant la coopération avec la feuille continue, **caractérisé en ce que** :
les trous (9) ont des premier (17) et second (18) groupes de dispositifs de distribution de dépression qui sont associés et tels que :
le premier groupe (17) peut être raccordé exclusivement au premier dispositif générateur d'une dépression (V1) de manière que les trous respectifs (9) délimitent exclusivement la première région de vide, et
le second groupe (18) peut être raccordé à la fois au premier dispositif (V1) ainsi qu'au second dispositif (V2) générateur de dépression, si bien que les trous respectifs (9) peuvent appartenir à la fois à la première région de vide ainsi qu'à la seconde région de vide suivant la position atteinte par l'élément mobile.

2. Dispositif selon la revendication 1, **caractérisé en ce que** l'élément mobile (1) comporte un tambour rotatif (7, 11, 15) ayant une surface percée de revêtement (14).

3. Dispositif selon la revendication 2, **caractérisé en ce que** le dispositif de commande comprend des orifices de distribution (20, 21) qui s'étendent le long de trajets angulaires qui sont au moins partiellement séparés le long du trajet de rotation du tambour (7, 11, 15).

4. Dispositif selon la revendication 3, **caractérisé en ce que** les orifices de distribution comprennent au moins un orifice (21) raccordé au second dispositif générateur de pression (V2) et qui s'étend le long d'un trajet angulaire ayant une extrémité qui correspond au raccord de la seconde région de vide avec le second dispositif générateur de dépression (V2), ladite extrémité étant à une position atteinte par le tambour (7, 11, 15) au moment auquel le dispositif de coupe (1) exécute l'intervention de coupe.

5. Dispositif selon la revendication 1 et la revendication 2, **caractérisé en ce que** :
les trous (9) s'étendent en direction pratiquement axiale par rapport au tambour rotatif associé (2), et
le dispositif de distribution de vide des premier et second groupes est constitué par des orifices d'extrémité (17, 18) du tambour (7, 11, 15), les orifices délimitant le premier groupe (17) de dispositifs de distribution se trouvant à une distance radiale déterminée de l'axe principal (X2) du tambour (7, 11, 15) alors que les orifices délimitant le second groupe (18) de dispositifs de distribution s'étendent radialement sur une certaine distance par rapport à l'axe principal (X2) du tambour (7, 11, 15).

6. Dispositif selon la revendication 3 et la revendication 5, **caractérisé en ce que** :
le premier orifice de distribution (20) s'étend pratiquement à la première distance radiale de l'axe principal du tambour (7, 11, 15), la première distance étant pratiquement égale à la distance radiale déterminée à laquelle les premiers orifices (17) sont disposés,
le second orifice de distribution (21) s'étend, par rapport à l'axe principal (X2) du tambour, à une seconde distance radiale différente de la première distance radiale, et
les orifices (18) délimitant le second groupe de dispositifs de distribution s'étendent radialement par rapport au tambour (7, 11, 15) au moins sur la partie comprise entre les première et seconde distances radiales.

7. Dispositif selon l'une quelconque des revendications 1, 6 et 7, **caractérisé en ce que** les premier (17) et second (18) groupes de dispositifs de distribution sont disposés dans au moins un flasque terminal (15) associé au tambour (7, 11, 15).

8. Dispositif selon la revendication 3 et la revendication 7, **caractérisé en ce que** le flasque terminal au moins (15) est fixé en rotation avec le tambour (7, 11, 15) alors que les premier (20) et second (21) orifices de distribution sont disposés dans au moins un élément de distribution (19) ayant une position statique par rapport au tambour (7, 11, 15).

9. Dispositif selon la revendication 8, **caractérisé en ce que** :
un prolongement axial de support au moins (10) qui tourne autour de l'axe principal (X2) est associé au tambour rotatif (7, 11, 15), et
l'élément de distribution au moins (19) a un creux pour le passage du prolongement axial au moins (10).

10. Dispositif selon la revendication 8 ou 9, **caractérisé en ce que** le flasque au moins (12) est formé d'un matériau pneumatique d'étanchéité qui peut former un joint étanche avec l'élément de distribution au moins (19).

11. Dispositif selon l'une quelconque des revendications précédentes, **caractérisé en ce que** le dispositif de coupe (1) comporte un élément rotatif de coupe dont le mouvement de rotation est synchronisé sur le mouvement de l'élément mobile (2).
